# EUROPEAN PATENT SPECIFICATION

(11) **EP 3 917 477 B1**
(45) Date of publication and mention of the grant of the patent: **20.03.2024**
(21) Application number: 20749118.4
(22) Date of filing: 30.01.2020
(51) Int. Cl.: A61F 13/15, A61F 13/514

(54) **FORMING STRUCTURE, APPARATUS AND METHOD FOR FORMING A BREATHABLE, LIQUID IMPERMEABLE, APERTURED FORMED FILM AND FILM MANUFACTURED THEREBY**
FORMSTRUKTUR, VORRICHTUNG UND VERFAHREN ZUR HERSTELLUNG EINER ATMUNGSFÄHIGEN, FLÜSSIGKEITSUNDURCHLÄSSIGEN, GELOCHTEN GEFORMTEN FOLIE UND SO HERGESTELLTE FOLIE
STRUCTURE DE FORMATION, APPAREIL ET PROCÉDÉ DE FORMATION D'UN FILM FORMÉ PERFORÉ, IMPERMÉABLE AUX LIQUIDES ET RESPIRANT, ET FILM AINSI FABRIQUÉ

(30) Priority: 01.02.2019 US 201962800255 P
(43) Date of publication of application: 08.12.2021
(73) Proprietor: Fitesa Film Products LLC, Simpsonville, SC 29681 (US)
(72) Inventor: MASCHINO, Andrew D., Paris, Illinois 61944 (US); GROSS, Alvin W., Richmond, Virginia 23225 (US); THOMAS, Paul E., Terre Haute, Indiana 47802 (US); SKOCHDOPOLE, Todd R., Moseley, Virginia 23120 (US)
(74) Representative: van Wijk, Alexander Pieter
(86) International application number: PCT/US2020/015881
(87) International publication number: WO 2020/160271

(56) References cited:
- EP-A1- 1 040 802
- WO-A1-01/87215
- US-A- 5 562 932
- US-A- 5 591 510
- US-A- 5 731 061
- US-B1- 6 570 059
- US-B2- 6 989 187
- US-B2- 7 291 763

## Description

### FIELD

The present invention is directed to a forming structure and an apparatus for forming a breathable, liquid impermeable, apertured formed film, a method for forming a breathable, liquid impermeable, apertured formed film, and a breathable, liquid impermeable, apertured formed film.

### BACKGROUND

A variety of well-known absorbent articles are configured to absorb body fluids. Examples of such absorbent articles include, but are not limited to, feminine hygiene products, such as sanitary napkins, baby diapers, and adult incontinence products. A typical absorbent article is generally constructed with a fluid permeable user-facing topsheet, which may be a three dimensional apertured polymer film or a nonwoven web or a polymer film/nonwoven laminate, an absorbent core, and a fluid impermeable garment or outwardly-facing backsheet, which may be a solid polymer film, for example. For some absorbent articles, it is desirable for the backsheet to be "breathable" so that moisture vapors may pass through the backsheet and potentially improve comfort to the wearer or user of the absorbent article. Breathability may be achieved by incorporating fillers into the backsheet that create micropores that are large enough to allow moisture vapor to pass therethrough, but small enough to prevent liquids from passing therethrough.

Attempts have been made to include three-dimensional apertures in the backsheet that are angled so that there is no line of sight through the backsheet. Such structures are disclosed in, for example, EP 1 040 802 A1, U.S. Patent Nos. 5,591,510, 6,413,247 and 6,570,059, as well as U.S. Patent Application Publication No. 2002/0133132. The three-dimensional apertures are designed to stay open when no pressure is applied to them, such as when the user is standing up, and to close when pressure is applied to them, such as when the user sits down. The larger apertures provide increased breathability, as compared to backsheets with micropores, when open. In EP 1 040 802 A1, capillaries are disclosed that have openings which have the shape of a single circle. Moreover, U.S. patent No. 5,591,510 discloses openings that are generally circular in shape, although also other shapes can be used. The openings in the outer surfaces in both EP 1 040 802 A1 and U.S. Patent No. 5,591510 differ considerably from openings used in accordance with the present invention. It has been found that three-dimensional apertures of the prior art may not prevent liquid from passing through the backsheet when the user stands up and the apertures re-open. This is due to the location of the opening of the three-dimensional aperture as compared to the level of the liquid being held by the absorbent article. If the structure of the three-dimensional aperture closes at a level below the level of the liquid, then when the structure of the three-dimensional aperture re-opens when pressure has been taken off of the structure, the structure may act as a pump and pull the liquid through the backsheet, which is undesirable.

It is desirable to have a backsheet for an absorbent article that allows moisture vapors to pass through, but not allow liquids to pass through.

### SUMMARY OF THE INVENTION

The present invention provides a forming structure as defined in claim 1. According to an aspect of the invention, there is provided a forming structure for forming a breathable, liquid impermeable, apertured formed film. The forming structure has an outer surface configured to receive a polymer web, an inner surface, a thickness between the outer surface and the inner surface, and a plurality of openings extending through the thickness at an acute angle relative to a plane tangential to the outer surface. Each of the plurality of openings has a shape at the outer surface defined by a first ellipse having a first major axis and a first minor axis and a second ellipse having a second major axis and a second minor axis, the first major axis is substantially parallel to a machine direction and coincides with the second minor axis. The second major axis is substantially parallel to a transverse direction orthogonal to the machine direction and offset from the first minor axis.

In an embodiment, the acute angle is between about 15° and about 45°. In an embodiment, the acute angle is between about 20° and about 40°.

In an embodiment, centers of adjacent openings are spaced equidistant from each other.

In an embodiment, each of the plurality of openings has a shape at the inner surface the same as the shape at the outer surface.

In an embodiment, the thickness of the forming structure comprises a plurality of layers, with each layer staggered relative to an adjacent layer to define the plurality of openings through the thickness at the acute angle.

According to an aspect of the present invention, there is provided an apparatus for forming a breathable, liquid impermeable, apertured formed film. The apparatus includes a forming structure having an outer surface configured to receive a polymer web, an inner surface, a thickness between the outer surface and the inner surface, and a plurality of openings extending through the thickness at an acute angle relative to a plane tangential to the outer surface. Each of the plurality of openings has a shape at the outer surface defined by a first ellipse having a first major axis and a first minor axis, and a second ellipse having a second major axis and a second minor axis. The first major axis is substantially parallel to a machine direction and coincides with the second minor axis. The second major axis is substantially parallel to a transverse direction orthogonal to the machine direction and offset from the first minor axis. The apparatus also includes a vacuum system configured to create a vacuum at the inner surface of the forming structure and pull portions of the polymer web into the plurality of openings.

According to an aspect of the invention, there is provided a method for forming a breathable, liquid impermeable, apertured formed film. The method includes contacting a polymer web with a forming structure. The forming structure has an outer surface configured to receive a polymer web, an inner surface, a thickness between the outer surface and the inner surface, and a plurality of openings extending through the thickness at an acute angle relative to a plane tangential to the outer surface. Each of the plurality of openings has a shape at the outer surface defined by a first ellipse having a first major axis and a first minor axis, and a second ellipse having a second major axis and a second minor axis. The first major axis is substantially parallel to a machine direction and coincides with the second minor axis. The second major axis is substantially parallel to a transverse direction orthogonal to the machine direction and offset from the first minor axis. The method also includes applying a vacuum to the inner surface of the forming structure to draw portions of the polymer web into the plurality of openings and create a plurality of apertured protuberances in the breathable, liquid impermeable, apertured formed film.

According to an aspect of the invention, there is provided a breathable, liquid impermeable, apertured formed film. The film includes a first side and a second side opposite the first side. The first side includes a plurality of two-dimensional apertures separated by first land areas. Each of the plurality of two-dimensional apertures has a shape defined by a first ellipse having a first major axis and a first minor axis, and a second ellipse having a second major axis and a second minor axis. The first major axis is substantially parallel to a machine direction of the film and coincides with the second minor axis. The second major axis is substantially parallel to a transverse direction orthogonal to the machine direction and offset from the first minor axis. The second side includes a plurality of apertured protuberances extending at an acute angle from a second land area opposite the first land area and aligned with the plurality of two-dimensional apertures.

According to an aspect of the invention, there is provided a breathable, liquid impermeable, apertured formed film that includes a first side and a second side opposite the first side. The first side includes a first land area and a plurality of two-dimensional apertures, and the second side includes a plurality of apertured protuberances extending at an acute angle from a second land area opposite the first land area and aligned with the plurality of two-dimensional apertures. The breathable, liquid impermeable, apertured formed film has an Air Permeability of greater than 25 m³/m²/min, and a Liquid Impact of less than 10 g/m².

In an embodiment, the Air Permeability is less than 100 m³/m²/min. In an embodiment, the Air Permeability is less than 50 m³/m²/min.

In an embodiment, the plurality of apertured protuberances close when pressure is applied to the breathable, liquid impermeable, formed film.

In an embodiment, the Caliper of the breathable, liquid impermeable, formed film is reduced by less than 50% when pressure of 0.827 bar (12 psi) is applied to the breathable, liquid impermeable, formed film.

These and other aspects, features, and characteristics of the present invention, as well as the methods of manufacturing and functions of the related elements of structure and the combination of parts and economies of manufacture, will become more apparent upon consideration of the following description and the appended claims with reference to the accompanying drawings, all of which form a part of this specification. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

The components of the following figures are illustrated to emphasize the general principles of the present disclosure and are not necessarily drawn to scale. Reference characters designating corresponding components are repeated as necessary throughout the figures for the sake of consistency and clarity.
Figure 1 is a schematic illustration of an absorbent article that includes embodiments of the present invention;
Figure 2 is a schematic illustration of an apparatus for manufacturing a film in accordance with embodiments of the present invention;
Figure 3 is a schematic illustration of an apparatus for manufacturing a laminate in accordance with embodiments of the invention;
Figure 4A is an illustration of a portion of a forming structure according to embodiments of the invention that may be used in the apparatus of Figure 2 and Figure 3;
Figure 4B is an illustration of a single opening of the forming structure of Figure 4A;
Figure 4C is a schematic illustration of a cross-section of a portion of the forming structure of Figure 4A;
Figure 5A is an illustration of a plan view of a first side of a film formed on the forming structure of Figure 4A;
Figure 5B is an illustration of a plan view of a second side of the film of Figure 5A;
Figure 5C is an illustration of a partial side view of the second side of the film of Figure 5B;
Figure 5D is an enlarged illustration of a side view of the second side of the film of Figure 5B;
Figure 6A is an illustration of a portion of a forming structure according to embodiments of the invention that may be used in the apparatus of Figure 2 and Figure 3;
Figure 6B is an illustration of a single opening of the forming structure of Figure 6A;
Figure 7A is an illustration of a plan view of a first side of a film formed on the forming structure of Figure 6A;
Figure 7B is an illustration of a plan view of a second side of the film of Figure 7A;
Figure 7C is an illustration of a partial side view of the second side of the film of Figure 7B;
Figure 7D is an enlarged illustration of a side view of the second side of the film of Figure 7B;
Figure 8A is an illustration of a plan view of a first side of a film made in accordance with an embodiment of the invention;
Figure 8B is an illustration of a plan view of a second side of the film of Figure 8A;
Figure 8C is an illustration of a partial side view of the second side of the film of Figure 8B;
Figure 9A is an illustration of a plan view of a first side of a film made in accordance with an embodiment of the invention;
Figure 9B is an illustration of a plan view of a second side of the film of Figure 9A;
Figure 9C is an illustration of a partial side view of the second side of the film of Figure 9B;
Figure 10A is an illustration of an aperture protuberance of a comparative film with no pressure being applied to the aperture protuberance;
Figure 10B is an illustration of the apertured protuberance of Figure 10A with some pressure being applied to the apertured protuberance;
Figure 10C is an illustration of the apertured protuberance of Figure 10B with additional pressure being applied to the apertured protuberance;
Figure 11A is an illustration of an apertured protuberance of a film according to an embodiment of the invention in an open position with no pressure being applied to the apertured protuberance;
Figure 11B is an illustration of the apertured protuberance of Figure 11AB in a closed position with pressure being applied to the apertured protuberance;
Figure 12A is an illustration of the apertured protuberance of Figure 11A with various dimensions depicted;
Figure 12B is an illustration of the apertured protuberance of Figure 11B with various dimensions depicted;
Figure 13A is an illustration of an apertured protuberance of a film according to an embodiment of the invention in an open position with no pressure being applied to the apertured protuberance; and
Figure 13B is an illustration of the apertured protuberance of Figure 13A in a closed position with pressure being applied to the apertured protuberance.

### DETAILED DESCRIPTION

### Glossary

As used herein, the expression "absorbent articles" denote articles that absorb and contain body fluids and other body exudates. More specifically, an absorbent article includes garments that are placed against or in proximity to the body of a wearer to absorb and contain the various exudates discharged from a body. Non-limiting examples of absorbent articles include, but are not limited to feminine hygiene products, baby diapers, adult incontinence products, and bandages.

Throughout this description, the term "web" refers to a material capable of being wound into a roll. Webs can be film webs, nonwoven webs, laminate webs, apertured laminate webs, etc. The face of a web refers to one of its two dimensional surfaces, as opposed to one of its edges.

The term "composite web" or "composite material" refers to a web that comprises two or more separate webs that are attached to each other in a face to face relationship. The attachment can be through the edges of the component webs, although the component webs lie in a face to face relationship with each other, or the attachment can be at particular spot locations across the component webs, or the attachment can be continuous.

The term "film" or "polymer film" in this description refers to a web made by extruding a molten curtain or sheet of thermoplastic polymeric material by a cast or blown extrusion process and then cooling the sheet to form a solid polymeric web. Films can be monolayer films, coextruded films, coated films, and composite films.

"Composite films" are films comprising more than one film where the at least two films are combined in a bonding process. Bonding processes may incorporate adhesive layers between the film layers.

Throughout this description, the expression "apertured films" denotes films that have a plurality of holes that extend from a first surface of the film to a second surface of the film.

A "two-dimensional apertured film" is a film in which no three-dimensional structure exists in the holes, which then connect the second surface of a flat film to the first surface of the film.

A "formed film" or a "three-dimensional film" is a film with protuberances, protrusions, or extended cells extending from at least one side thereof, and an "apertured formed film" or a "three-dimensional apertured film" is a film in which a three-dimensional structure exists in the apertures (e.g., the apertures have a depth that is thicker than the thickness of the film), or the protuberances or protrusions or extended cells have apertures therethrough.

The term "protuberance" as used herein refers to a three-dimensional member comprising an apertured base portion located in the plane of the first surface of the film and a sidewall portion extending generally in the direction of the second surface of the film. Each base portion has an associated sidewall portion. Sidewall portions terminate in "distal ends" located in the plane of the second surface of the film. The ends of the protuberances may be apertured or unapertured.

"Apertured protuberance" as used herein refers to a protuberance that has an aperture at its base portion or proximal end in the plane of the second surface, as well as its distal or protubered end. The apertures in the base portions of the protuberances, also called "primary apertures," may be in the shape of polygons, for example squares, hexagons, pentagons, ellipses, circles, ovals, or slots, or may have other shapes, in a regulated or random pattern. Additional shapes according to illustrated embodiments of the invention are described in further detail below. The apertured distal or protubered ends are called "secondary apertures," and may be in the shape of polygons, such as squares, hexagons, pentagons, ellipses, circles, ovals, slots, or may be in other shapes. The sidewall portion of the apertured protuberance extends from the primary aperture to the secondary aperture.

The term "nonwoven" means a web comprising a plurality of fibers. The fibers may be bonded to each other or may be unbonded. The fibers may be staple fibers or continuous fibers or filaments. The fibers may comprise a single material or may comprise a multitude of materials, either as a combination of different fibers or as a combination of similar fibers with each comprised of different materials.

As used herein, "nonwoven web" is used in its generic sense to define a generally planar structure that is relatively flat, flexible and porous, and includes staple fibers or continuous fibers or filaments. The nonwoven web may be the product of any process for forming the same, such as nonwoven spunbond and melt blown nonwoven webs. The nonwoven web may include a composite or combination of webs. The nonwoven web may comprise any polymeric material from which a fiber can be produced and/or may comprise cotton or other natural fibers. In an embodiment, the nonwoven web may be a spunbond material, made of polypropylene fiber. Fibers that comprise different polymers may also be blended. In an embodiment, the fibers may be so-called bi-component ("bi-co") fibers that comprise a core of one material and a sheath of another material.

The term "forming structure" as used herein refers to a three-dimensional molding apparatus that comprises indentations or openings used to form protuberances, extended cells or apertures in films, or protuberances in nonwoven webs. In an embodiment, forming structures comprise tubular members, having a width and a diameter. In alternative embodiments, forming structures may comprise belts having a width and a length. The transverse direction is the direction parallel to the width of the forming structure. The machine direction is the direction parallel to the direction of rotation of the forming structure, and is perpendicular to the transverse direction.

### Test Methods

Caliper, which may also be referred to as loft or thickness, is measured generally following ASTM-D645 using a motorized micrometer having a 5.08 cm (2-inch) diameter anvil and dead weight load of 14.72 g/cm² (95 g/in²), and using a dwell time of 2-5 seconds. Results may be reported in mils or micrometers (µm).

Air Permeability is measured in a TEXTEST FX3300 Air Permeability Tester, which measures the volume of air that passes through a test sample per minute and is recorded in cubic meters of air per square meter (of test sample) per minute (m³/m²/min).

Liquid Impact is measured with an apparatus similar to the device described in U.S. Patent No. 5,865,823 and associated method for measuring the dynamic fluid impact value of a sample after a single insult. Results are reported below in grams per square meter (g/m²).

### Description of Embodiments of the Invention

Various embodiments of the present invention will now be highlighted. The discussion of any one embodiment is not intended to limit the scope of the present invention. To the contrary, aspects of the embodiments are intended to emphasize the breadth of the invention, whether encompassed by the claims or not. Furthermore, any and all variations of the embodiments, now known or developed in the future, also are intended to fall within the scope of the invention.

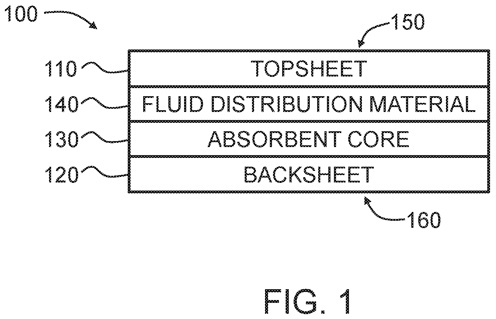

Figure 1 schematically illustrates an absorbent article 100 that includes embodiments of the invention. As illustrated, the absorbent article 100 includes a topsheet 110, a backsheet 120, and an absorbent core 130 positioned in between the topsheet 110 and the backsheet 120. The absorbent article 100 also includes a fluid distribution material 140 positioned in between the topsheet 110 and the absorbent core 130. The absorbent article 100 has a first side 150 that is user or person facing, and a second side 160 that is garment facing when in use, i.e., when being worn by the user.

The topsheet 110, which may be in the form of a two-dimensional or three-dimensional apertured film, a nonwoven web, or a laminate of an apertured film and a nonwoven web, is permeable to fluids and is configured to face the user wearing the absorbent article 100 and contact the user's skin. The topsheet 110 receives insults of fluid from the user, and the fluid passes through the topsheet 110 to the fluid distribution material 140. The fluid distribution material 140 is also permeable and is configured to receive the fluid from the topsheet 110 and distribute the fluid to the absorbent core 130. The absorbent core 130, which includes absorbent materials, receives the fluid from the fluid distribution material 140 and stores the fluid until the absorbent article 100 is discarded.

The backsheet 120, discussed in further detail below, may be in the form of a polymer film or a laminate of a polymer film and nonwoven web, prevents liquid and other body exudates from leaking out of the bottom side of the absorbent core 130. The backsheet 120 may be breathable so that air and moisture vapor, but not liquid, may pass through.

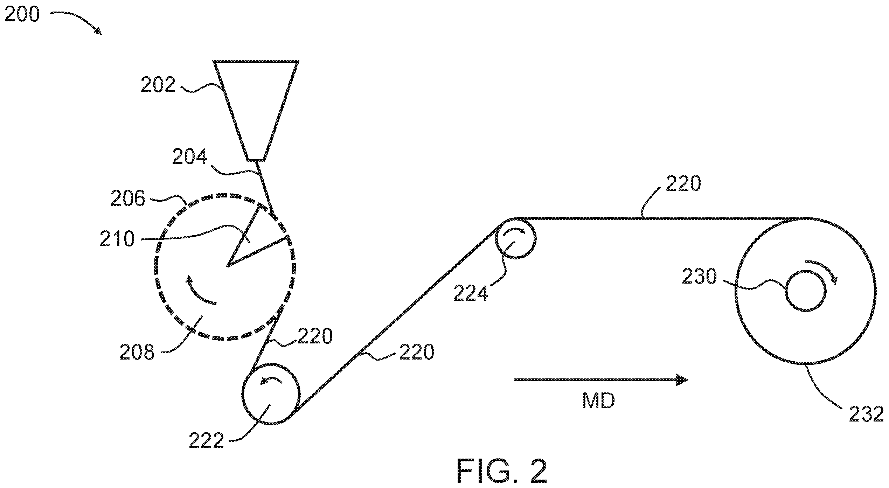

Figure 2 schematically illustrates an apparatus 200 that may be used to manufacture the backsheet 120 in accordance with embodiments of the invention described herein. As illustrated, an extrusion die 202 extrudes a polymer web 204 onto a forming structure 206 that rotates about a cylinder 208 that has a vacuum slot 210 through which a vacuum is pulled. The polymer web 204 may include, for example, one or more polyolefin materials, including but not limited to polyethylene, ultra-low density polyethylene, low density polyethylene, linear low density polyethylene, linear medium density polyethylene, high density polyethylene, polypropylene, ethylene-vinyl acetates, metallocene, as well as other polymers. The polymer web 204 may include one or more elastomeric polymers, including but not limited to polypropylene based elastomers, ethylene based elastomers, copolyester based elastomers, olefin block copolymers, styrenic block copolymers and the like, or combinations thereof. Additives, such as surfactants, fillers, colorants, opacifying agents and/or other additives known in the art may also be used in the polymer web 204.

As the polymer web 204 crosses over the vacuum slot 210 apertured protuberances are formed in the polymer web 204 (i.e., the solidified melt curtain) in substantially the same pattern that is provided by the forming structure 206. As the polymer web 204 is apertured, air flow is initiated through the apertured protuberances which cools and solidifies the apertured protuberances. The polymer web 204 is also cooled by the forming structure 206. The resulting vacuum formed film 220 is pulled off of forming structure 206 by a peel roller 222 and travels across one or more subsequent rollers 224 until it may be wound by a winder 230 into a roll 232. Additional rollers and/or other pieces of equipment may be used in the apparatus 200.

The illustrated embodiment is not intended to be limiting in any way. For example, in an embodiment, the apparatus 200 may also include additional equipment, such as a corona treatment apparatus, printers, festooning equipment, spooling equipment, and additional processing equipment that may emboss the vacuum formed film 220.

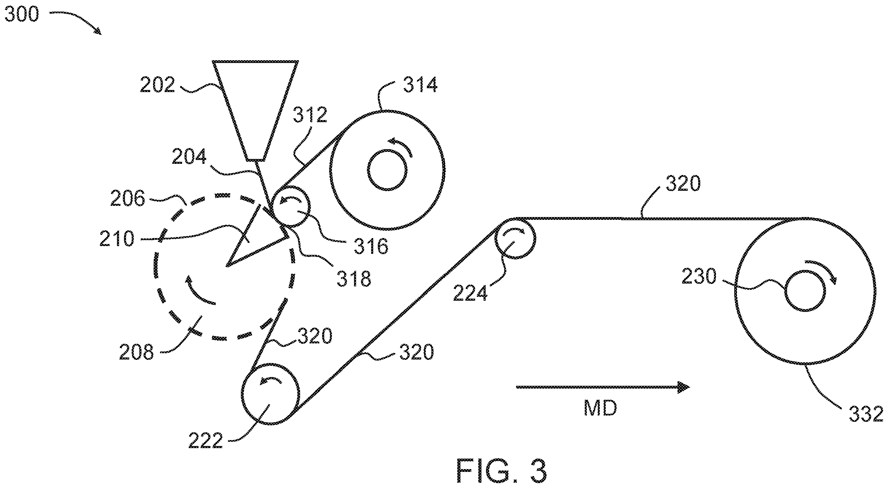

Figure 3 schematically illustrates an apparatus 300 that may be used to manufacture the backsheet 120 of embodiments of the invention described herein. As illustrated, the apparatus 300 is substantially the same as the apparatus 200 depicted in Figure 2, but also includes a nonwoven web 312 that is unwound from a roll 314 over a laminating roller 316 and directed to the polymer web 204 while the polymer web 204 is still molten at an impingement point 318 between the rotating forming structure 206 and the laminating roller 316.

The fibers of the nonwoven web 312 adjacent to the polymer web 204 embed in the surface of the polymer web 204 as the two layers cross over the vacuum slot 210 together, where apertured protuberances are formed in the polymer web 204 in substantially the same pattern that is provided by the forming structure 206. As the polymer web 204 is apertured, air flow is initiated through the apertured protuberances which cools and solidifies the apertured protuberances. The polymer web is also cooled by the forming structure 206 as the fibers of the nonwoven web 312 are embedded in land areas between the apertured protuberances so that the nonwoven is bonded to the formed film layer at the land areas. The resulting vacuum formed composite web 320 is pulled off of forming structure 206 by the peel roller 222 and travels to the one or more subsequent rollers 224 until it may be wound by the winder 230 into a roll 332. Additional rollers and/or other pieces of equipment may be used in the apparatus 300.

The illustrated embodiment is not intended to be limiting in any way. For example, in an embodiment, the apparatus 300 may also include additional equipment, such as a corona treatment apparatus, printers, festooning equipment, spooling equipment, and additional processing equipment that may emboss the vacuum formed laminate 320.

In an embodiment, instead of extruding the polymer web 204 in the form of a melt curtain directly onto the forming structure 206, an already formed solid polymer web may be unwound from a roll, reheated to soften the solid polymer web, and fed to the forming structure 206 so that the apertured protuberances may be formed in the polymer web. The polymer web may include a single layer of material or may include multiple layers of material.

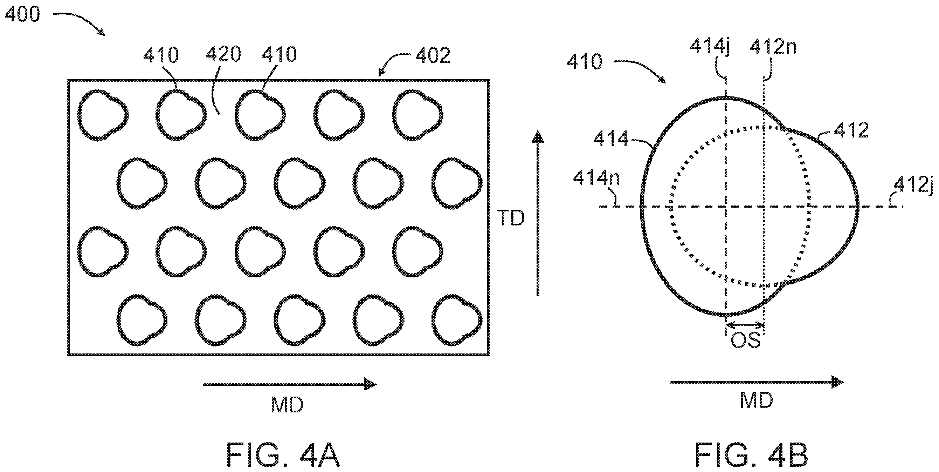

Figure 4A illustrates a portion of a top or outer surface 402 of a forming structure 400 that may be used as the forming structure 206 of the apparatus 200, 300 of Figures 2 and 3 in accordance with an embodiment of the invention. As illustrated, the forming structure 400 includes a plurality of openings 410 and a land area 420 in between the openings 410. The land area 420 is essentially the solid portion of the forming structure 400. The plurality of openings 410 are oriented relative to a machine direction MD of the apparatus 200 and 300 described above in the manner shown. A transverse direction TD, which is perpendicular or orthogonal to the machine direction MD, is also shown in Figure 4A. In the illustrated embodiment, centers of adjacent openings 410 are spaced equidistant to each other. Other arrangements of and spacing between the openings 410 are contemplated as being within the scope of the invention. The illustrated embodiment is not intended to be limiting in any way.

Figure 4B illustrates a single opening 410 in greater detail. As illustrated, the shape of the opening 410 at the top surface 402 of the forming structure 400 can be described by a first ellipse 412 and a second ellipse 414 that overlap with one another. The first ellipse 412 has a major axis 412j that is substantially parallel to the machine direction MD and a minor axis 412n that is substantially parallel to the transverse direction TD. The second ellipse 414 has a major axis 414j that is substantially parallel to the transverse direction TD and a minor axis 414n that is substantially parallel to the machine direction MD and coincides with the major axis 412j of the first ellipse 412. In addition, the major axis 414j of the second ellipse 414 is spaced from the minor axis 412n of the first ellipse 412 by an offset OS in the machine direction MD, as illustrated.

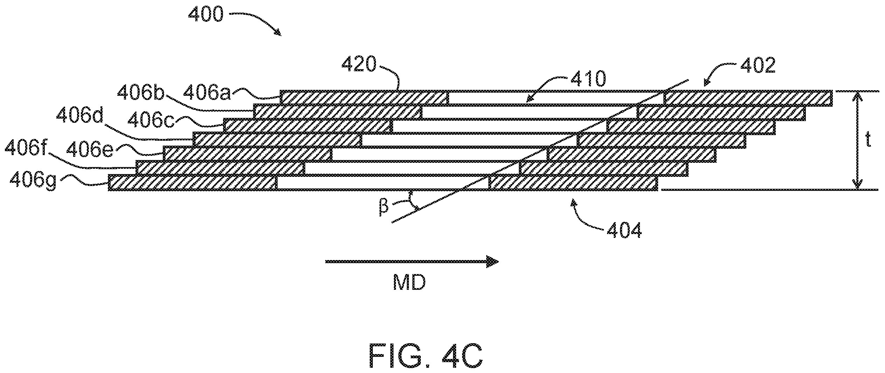

Figure 4C illustrates a partial cross-section of the forming structure 400. As illustrated the forming structure 400 also includes an inner surface 404, which contacts the rotating cylinder 208 described above. The forming structure 400 has a thickness t between the outer surface 402 and the inner surface 404, and is made up of a plurality of layers 406a-406g. Although seven layers 406a-406g are illustrated in Figure 4C, more or less layers may be used. For example, in an embodiment up to twenty layers or more may be used to create the forming structure 400. Each layer is staggered relative to an adjacent layer in a manner that defines the opening 410 at an angle β. In an embodiment, the angle β may be between about 15° and about 45°. In an embodiment, the angle β may between about 20° and about 40°. In an embodiment, the angle β may be about 25° or about 30° or about 35°. In an embodiment, the forming structure 400 may be made from a single, thicker layer of material. The illustrated embodiment is not intended to be limiting in any way.

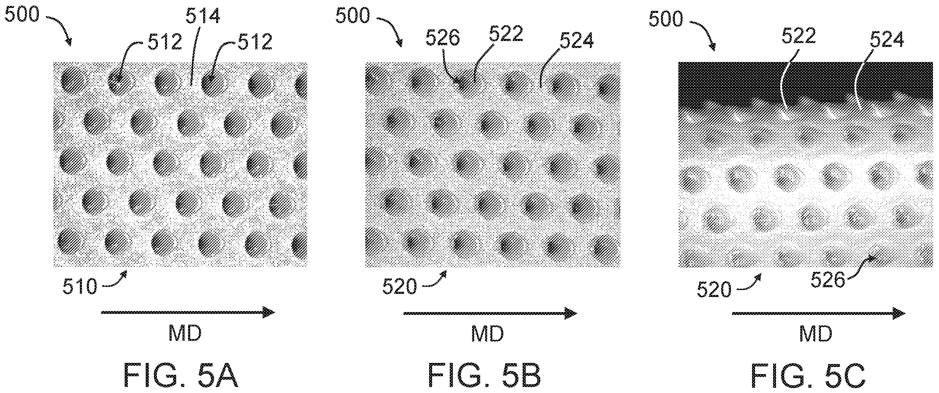

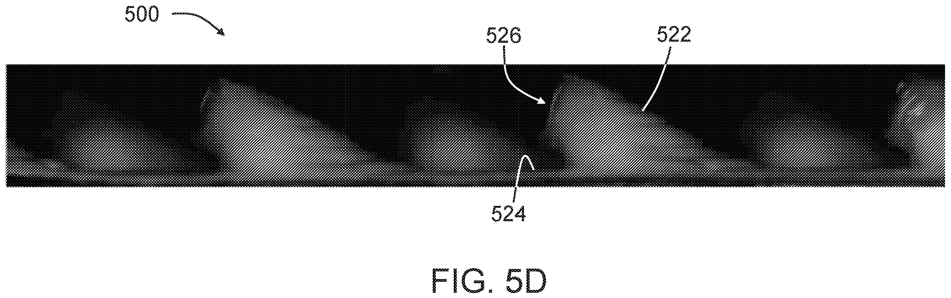

Figure 5A illustrates a first side 510, which may also be referred to as the female side, of a film 500 according to an embodiment of the invention. The film 500 was formed with the forming structure 400 illustrated in Figures 4A and 4C and included a blend of polyolefins and white colorant. The first side 510 of the film 500 includes a plurality of two-dimensional apertures 512 that are separated by a land area 514. Figure 5B illustrates a second side 520, which may also be referred to as the male side, of the film 500. The second side 520 includes a plurality of protuberances 522 that are separated by a land area 524. The land area 524 of the second side 520 is opposite the land area 514 of the first side 510. Each of the plurality of protuberances 522 includes an aperture 526 at a distal end thereof. Figures 5C and 5D illustrate the protuberances 522 in greater detail. As illustrated, the protuberances 522 extend from the land area 524 at an acute angle, which may be the same or similar to the angle β described above with respect to the forming structure 400. Additional attributes of the protuberances 522 are described in further detail below.

Films 500 that were made with the forming structure 400 and different blends of polyolefins at a target basis weight of 25 grams per square meter (gsm) were tested for Caliper, Air Permeability and Liquid Impact to determine suitability for use as a backsheet. The Caliper of the films were tested to be between about 336 µm and about 406 µm. The Air Permeability of the films were tested to be between about 27 m³/m²/min and about 41 m³/m²/min. The Liquid Impact of the films were tested to be between about 4 g/m² and about 10 g/m².

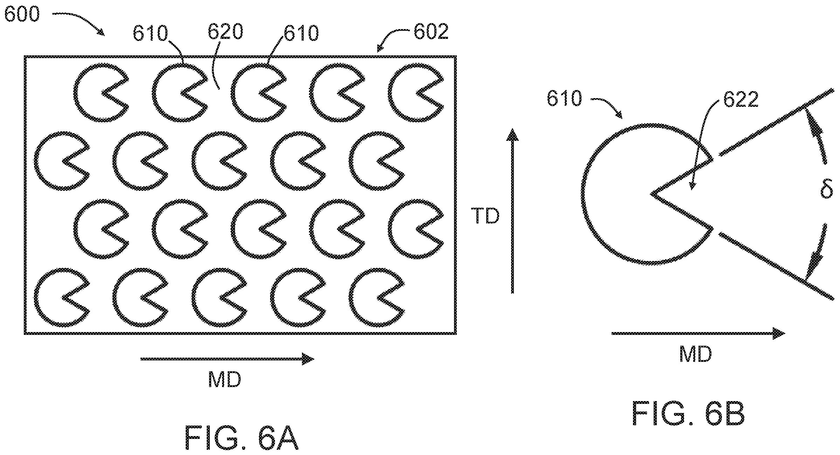

Figure 6A illustrates a portion of a top or outer surface 602 of a forming structure 600 that may be used as the forming structure 206 of the apparatus 200, 300 of Figures 2 and 3, according to an embodiment of the invention. As illustrated, the forming structure 600 includes a plurality of openings 610 and a land area 620 in between the openings 610. The land area 620 is essentially the solid portion of the forming structure 600. The plurality of openings 610 are oriented relative to a machine direction MD of the apparatus 200 and 300 described above in the manner shown. The transverse direction TD, which is perpendicular or orthogonal to the machine direction MD, is also shown in Figure 6A. In the illustrated embodiment, centers of adjacent openings 610 are spaced equidistant to each other. Other arrangements of and spacing between the openings 610 are contemplated as being within the scope of the invention. The illustrated embodiment is not intended to be limiting in any way.

Figure 6B illustrates a single opening 610 in greater detail. As illustrated, the opening 610 is substantially circular with the exception of a wedge portion 622 not cut out. The wedge portion 622 forms part of the land area 620 of the forming structure 600. The size of the wedge portion 622 may be defined by wedge angle δ. In the illustrated embodiment, the wedge angle δ is about 60°. The illustrated embodiment is not intended to be limiting in any way. In other embodiments, the wedge angle δ may be greater than or less than 60°. For example, the wedge angle δ may be in the range of 25° to 75°. Similar to the forming screen 400 illustrated in Figure 4C, the forming screen 600 may also include a plurality of layers that are stacked in a manner that provides the openings 610 with angled sidewalls.

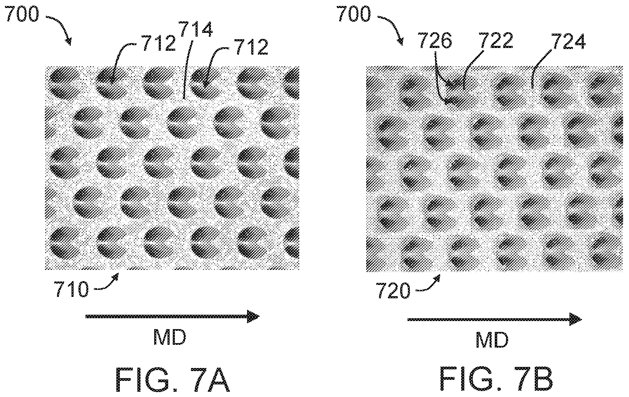

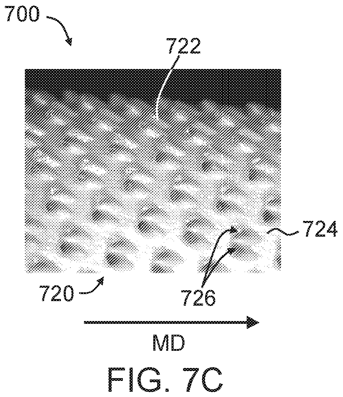

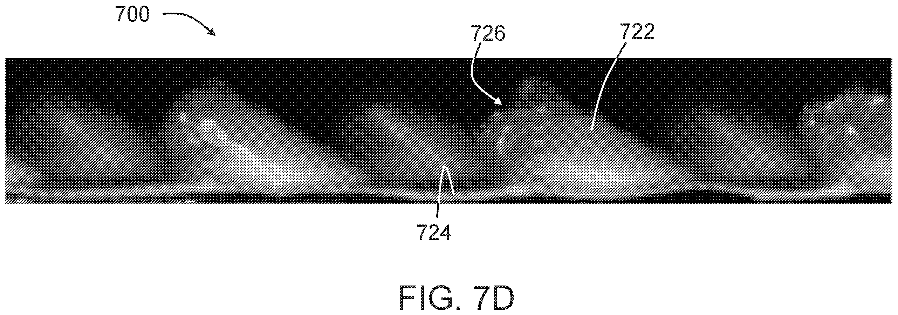

Figure 7A illustrates a first side 710, which may also be referred to as the female side, of a film 700 according to an embodiment of the invention. The film 700 was formed with the forming structure 600 illustrated in Figure 6A and included a blend of polyolefins and white colorant. The first side 710 of the film 700 includes a plurality of two-dimensional apertures 712 that are separated by a land area 714. Figure 7B illustrates a second side 720, which may also be referred to as the male side, of the film 700. The second side 720 includes a plurality of protuberances 722 that are separated by a land area 724. The land area 724 of the second side 720 is opposite the land area 714 of the first side 710. As illustrated, each of the plurality of protuberances 722 includes two apertures 726 at a distal end thereof. Figures 7C and 7D illustrate the protuberances 722 in greater detail. As illustrated, the protuberances 722 extend from the land area 724 at an angle, which may be the same or similar to the angle β described above with respect to the forming structure 400.

Films 700 that were made with the forming structure 600 and different blends of polyolefins at a target basis weight of 25 gsm were tested for Caliper, Air Permeability and Liquid Impact to determine suitability for use as a backsheet. The Caliper of the films were tested to be between about 357 µm and about 388 µm. The Air Permeability of the films were tested to be between about 30 m³/m²/min and about 34 m³/m²/min. The Liquid Impact of the films were tested to be between about 22 g/m² and about 60 g/m².

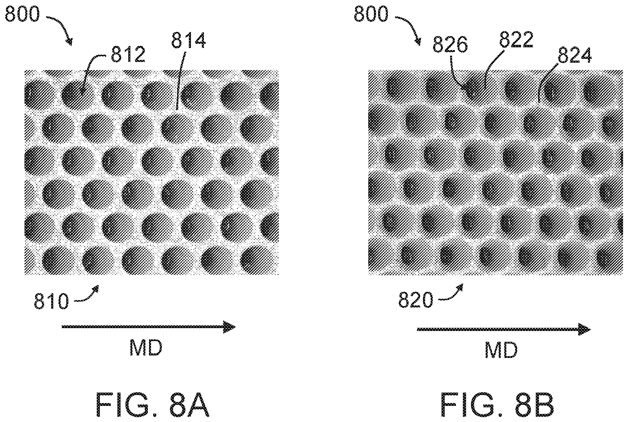

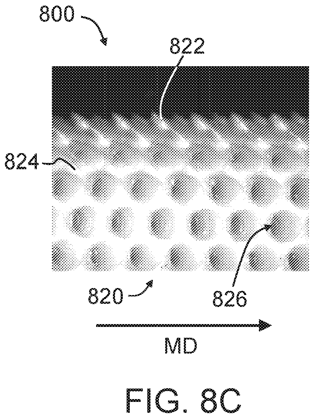

Figure 8A illustrates a first side 810, which may also be referred to as the female side, of a film 800 according to an embodiment of the invention. The first side 810 of the film 800 includes a plurality of two-dimensional apertures 812 that are separated by a land area 814. The film 800 was formed with a forming structure (not shown) having a plurality of apertures, with each aperture having a substantially circular shape and a diameter of about 1000 µm (40 mils). The centers of adjacent apertures of the forming structure were equidistant to one another and the apertures were arranged in a pattern of about 7.9 apertures per linear cm (20 apertures per linear inch), or "mesh" (about 7.9 apertures per linear centimeter). The film 800 was formed from a blend of polyolefins and white colorant. Figure 8B illustrates a second side 820, which may also be referred to as the male side, of the film 800. The second side 820 includes a plurality of protuberances 822 that are separated by a land area 824. The land area 824 of the second side 820 is opposite the land area 814 of the first side 810. Each of the plurality of protuberances 822 includes an aperture 826 at a distal end thereof. As illustrated in Figure 8C, the protuberances 822 extend from the land area 824 at an angle, which may be the same or similar to the angle β described above with respect to the forming structure 400.

Films 800 that were made with the above-described forming structure and different blends of polyolefins at a target basis weight of 25 gsm were tested for Caliper, Air Permeability and Liquid Impact to determine suitability for use as a backsheet. The Caliper of the films were tested to be between about 436 µm and about 465 µm. The Air Permeability of the films were tested to be between about 114 m³/m²/min and about 136 m³/m²/min. The Liquid Impact of the films were tested to be between about 15 g/m² and about 24 g/m².

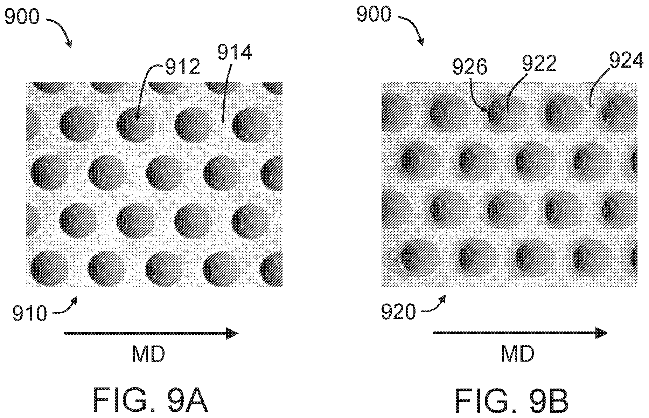

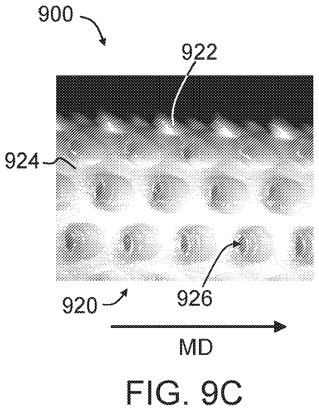

Figure 9A illustrates a first side 910, which may also be referred to as the female side, of a film 900 according to an embodiment of the invention. The first side 910 of the film 900 includes a plurality of two-dimensional apertures 912 that are separated by a land area 914. The film 900 was formed with a forming structure (not shown) having a plurality of apertures, with each aperture having a substantially circular shape and a diameter of about 1200 µm (48 mils) . The centers of adjacent apertures of the forming structure were equidistant to one another and the apertures were arranged in a pattern of about 5.5 apertures per linear centimeter (14 apertures per linear inch), or "mesh" (about 5.5 apertures per linear centimeter). The film 900 was formed from a blend of polyolefins and white colorant. Figure 9B illustrates a second side 920, which may also be referred to as the male side, of the film 900. The second side 920 includes a plurality of protuberances 922 that are separated by a land area 924. The land area 924 of the second side 920 is opposite the land area 914 of the first side 910. Each of the plurality of protuberances 922 includes an aperture 926 at a distal end thereof. As illustrated in Figure 8B, the protuberances 922 extend from the land area 924 at an angle, which may be the same or similar to the angle β described above with respect to the forming structure 400.

Films 900 that were made with the above-described forming structure and different blends of polyolefins at a target basis weight of 25 gsm were tested for Caliper, Air Permeability and Liquid Impact to determine suitability for use as a backsheet. The Caliper of the films were tested to be between about 429 µm and about 474 µm. The Air Permeability of the films were tested to be between about 79 m³/m²/min and about 93 m³/m²/min. The Liquid Impact of the films were tested to be between about 9 g/m² and about 22 g/m².

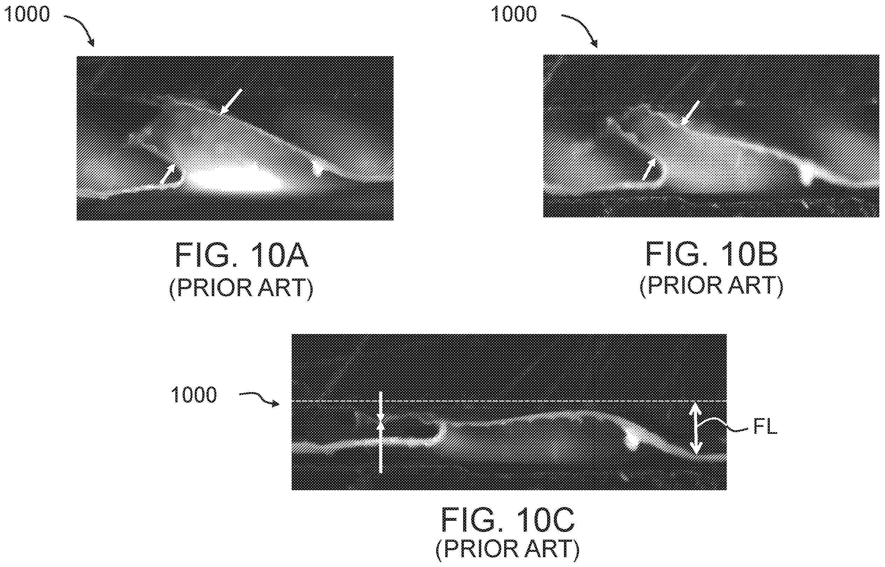

Figures 10A-10C illustrate an apertured protuberance 1000 of a comparative (prior art) film, which was not made in accordance with embodiments of the invention. Figure 10A illustrates the apertured protuberance 1000 in a fully open position with no pressure being applied to the apertured protuberance 1000, which may simulate a user wearing an absorbent article in a standing position, for example. With the apertured protuberance 1000 in the fully open position, air is able to enter the absorbent article and moisture vapors are able to exit the absorbent article. Figure 10B illustrates the same apertured protuberance 1000 of Figure 10A with some pressure being applied to the apertured protuberance 1000 from above the apertured protuberance 1000, which may simulate the user in the process of sitting down, for example. The pair of arrows in Figure 10A and the pair of arrows in Figure 10B are pointing to approximately the same locations on opposite sides of the apertured protuberance 1000. As illustrated, sidewalls of the apertured protuberance 1000 move closer together as pressure is applied.

Figure 10C illustrates the apertured protuberance 1000 with additional pressure being applied to the apertured protuberance 1000 from above, which may simulate the user sitting down, for example. The pairs of arrows shown on the left side of Figure 10C indicate the full closure of the apertured protuberance 1000, i.e., the opposing sidewalls of the apertured protuberance 1000 are in contact with each other in the region of the location of the pairs of arrows. If there is liquid in the absorbent core 130 of the absorbent article 100, the pressure being applied by the user while sitting down may cause the liquid to exit the absorbent core 130 and rest against the backsheet 120, as represented by the dashed line in Figure 10C and the double arrow, which represents the fluid level FL. Notably, the point of closure of the opposed sidewalls (represented by the pair of arrows on the left side of Figure 10C) is located below the fluid level FL. When the user stands up and the apertured protuberance 1000 reopens while being exposed to the liquid, the apertured protuberance 1000 may actually act as a pump by pulling the liquid therethrough and out of the absorbent article, which is undesirable.

The caliper or overall height of the apertured protuberance 1000 was measured when different pressures where applied to the apertured protuberance 1000. Specifically, the overall height of the apertured protuberance 1000 was measured at 0 bar (0 psi, height of 424 µm), 0.386 bar (5.6 psi,height of 279 µm), 0.483 bar (7 psi, height of 206 µm), and 0.827 bar (12 psi, height of 147 µm). At 0.386 bar (5.6 psi) applied pressure, the height decreased by about 34% (relative to the height at 0 bar (0 psi)), at 0.483 bar (7 psi), the height decreased by about 52% (relative to the height at 0 bar (0 psi)), and at 0.827 bar (12 psi)), the height decreased by about 65% (relative to the height at 0 bar (0 psi)).

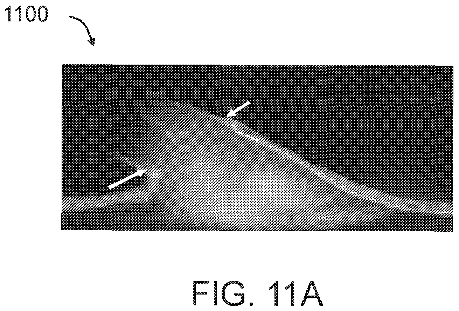

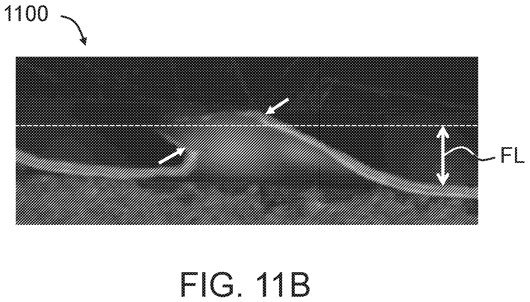

Figures 11A and 11B illustrate an apertured protuberance 1100 of a film that was made in accordance with embodiments of the invention with the forming structure 400 described above and may be used as a backsheet 120 for an absorbent article 100. Figure 11A illustrates the apertured protuberance 1100 in a fully open position with no pressure being applied to the apertured protuberance 1100. Figure 11B illustrates the apertured protuberance 1100 in a fully closed position with pressure being applied from above. The pair of arrows in Figure 11A and the pair of arrows in Figure 11B are pointing to approximately the same locations on opposite sides of the apertured protuberance 1100. As illustrated, the pair of arrows in Figure 11B remain apart, but the sidewalls of the apertured protuberance 1100 more distal to the pair of arrows contact each other to close the apertured protuberance 1100. The fluid level FL is at the same level as was illustrated in Figure 10C. Notably, the point at which the sidewalls meet to close the apertured protuberance 1100 is slightly above the fluid level. When the user stands up, the apertured protuberance 1100 is able to reopen above the fluid level FL so that liquid will not be pumped out of the absorbent article through the apertured protuberance 1100, which is desirable.

The caliper or overall height of the apertured protuberance 1100 was measured when different pressures where applied to the apertured protuberance 1100. Specifically, the overall height of the apertured protuberance 1100 was measured at 0 bar (0 psi, height of 370 µm), 0.386 bar (5.6 psi, height of 300 µm), 0.483 bar (7 psi, height of 262 µm), and 0.827 bar (12 psi, height of 234 µm). At 0.386 bar (5.6 psi) applied pressure, the height decreased by about 19% (relative to the height at 0 bar (0 psi)), at 0.483 bar (7 psi), the height decreased by about 29% (relative to the height at 0 bar (0 psi)), and at 0.827 bar (12 psi), the height decreased by about 37% (relative to the height at 0 bar (0 psi)).

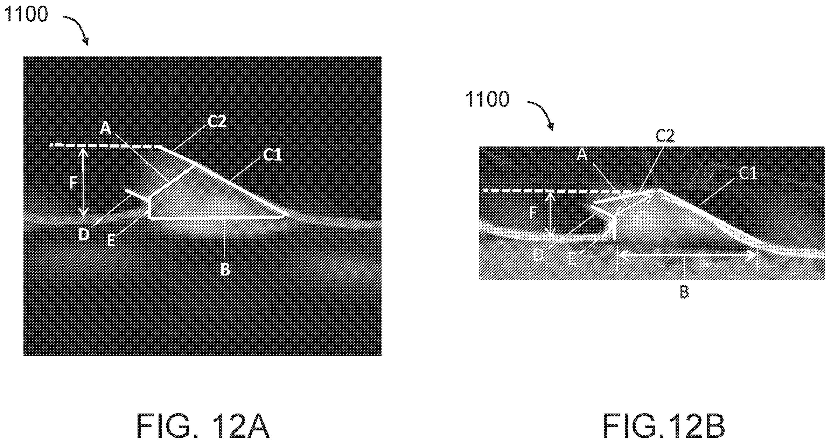

Figures 12A and 12B are the same images as Figures 11A and 11B with the addition of various dimensions of the apertured protuberance 1100 both in the open and closed positions. Table I below lists that various dimensions for both the open and closed positions.

**Table I: Dimensions of Open and Closed Apertured Protuberance 1100**

| **Dimension** | **Description** | **Open Value (µm)** | **Closed Value (µm)** |
|---|---|---|---|
| A | Width between sidewalls | 320 | 300 |
| B | Width of base opening | 813 | 813 |
| C1 | Length of thicker upper sidewall | 721 | 721 |
| C2 | Length of thinner upper sidewall | 295 | 295 |
| D | Length of thinner lower sidewall | 152 | 152 |
| E | Length of thicker lower sidewall | 132 | 132 |
| F | Height of apertured protuberance | 406 | 262 |

As indicated by the measurements listed in Table I, only the width between the sidewalls A and the height of the apertured protuberance 1100 changed under pressure. As illustrated in Figures 11A-12B, the thicker portions of the upper and lower sidewalls, as well as the thinner portion of the lower sidewall substantially remain in the same positions, while the thinner portion of the upper sidewall moves from a slanted, upward position to a substantially horizontal position, thereby causing the apertured protuberance 1100 to close.

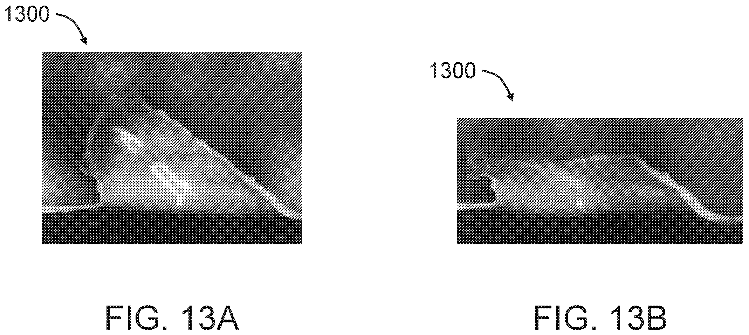

Figure 13A and 13B illustrate an apertured protuberance 1300 of the film 900 described above that was made in accordance with embodiments of the invention and may be used as a backsheet 120 for an absorbent article 100. Figure 13A illustrates the apertured protuberance 1300 in a fully open position with no pressure being applied to the apertured protuberance 1300. Figure 13B illustrates the apertured protuberance 1300 in a fully closed position with pressure being applied from above. The same measurements that were taken for the apertured protuberance 1100 of Figures 12A and 12B were also taken for the apertured protuberance 1300 of Figures 13A and 13B, and are summarized in Table II below.

**Table II: Dimensions of Open and Closed Apertured Protuberance 1300**

| **Dimension** | **Description** | **Open Value (µm)** | **Closed Value (µm)** |
|---|---|---|---|
| A | Width between sidewalls | 716 | 792 |
| B | Width of base opening | 1077 | 1092 |
| C1 | Length of thicker upper sidewall | 452 | 439 |
| C2 | Length of thinner upper sidewall | 914 | 907 |
| D | Length of thinner lower sidewall | 140 | 142 |
| E | Length of thicker lower sidewall | 146 | 145 |
| F | Height of apertured protuberance | 521 | 267 |

As indicated by the measurements listed in Table II and illustrated in Figures 13A and 13B, the application of pressure to the apertured protuberance 1300 caused the width between the sidewalls A to increase and the height F of the apertured protuberance 1300 to decrease. The lengths of the thinner D and thicker E lower sidewalls stayed approximately the same.

Although embodiments of the present invention were described above with respect to films formed with the forming structure 206, it should be understood that laminate or composite materials of the above-described films and nonwoven webs are also within the scope of the present invention. Such composite materials may be made using the apparatus 300 illustrated in Figure 3 and described above.

## Claims

1. A forming structure (400) having an outer surface (402) configured to receive a polymer web, an inner surface (404), a thickness (t) between the outer surface (402) and the inner surface (404), and a plurality of openings (410) extending through the thickness (t) at an acute angle (β) relative to a plane tangential to the outer surface (402), each of the plurality of openings (410) having a shape at the outer surface (402) defined by a first ellipse (412) having a first major axis (412j) and a first minor axis (412n), and a second ellipse (414) having a second major axis (414j) and a second minor axis (414n), the first major axis (412j) substantially parallel to a machine direction and coinciding with the second minor axis (414n), the second major axis (414j) substantially parallel to a transverse direction orthogonal to the machine direction and offset from the first minor axis (412n).

2. The forming structure (400) according to claim 1, wherein the acute angle (β) is between about 15° and about 45°.

3. The forming structure (400) according to claim 2, wherein the acute angle (β) is between about 20° and about 40°.

4. The forming structure (400) according to claim 1, wherein centers of adjacent openings (410) are spaced equidistant from each other.

5. The forming structure (400) according to claim 1, wherein each of the plurality of openings (410) has a shape at the inner surface (404) the same as the shape at the outer surface (402).

6. The forming structure (400) according to claim 1, wherein the thickness (t) of the forming structure comprises a plurality of layers, with each layer staggered relative to an adjacent layer to define the plurality of openings through the thickness (t) at the acute angle (β).

7. An apparatus (200) for forming a breathable, liquid impermeable, apertured formed film, the apparatus comprising:
the forming structure (400) according to any of claims 1-6; and
a vacuum system (210) configured to create a vacuum at the inner surface (404) of the forming structure and pull portions of the polymer web into the plurality of openings (410).

8. A method for forming a breathable, liquid impermeable, apertured formed film (400), the method comprising:
contacting a polymer web (204) with the forming structure (400) according to any of claims 1-6; and
applying a vacuum to the inner surface (404) of the forming structure to draw portions of the polymer web (204) into the plurality of openings (410) and create a plurality of apertured protuberances.

9. A breathable, liquid impermeable, apertured formed film (500), comprising:
a first side (510) and a second side (520) opposite the first side (510);
the first side (510) comprising a first land area (514) and a plurality of two-dimensional apertures (512), each of the plurality of two-dimensional apertures (512) having a shape defined by a first ellipse (412) having a first major axis (412j) and a first minor axis (412n), and a second ellipse (414) having a second major axis (414j) and a second minor axis (414n), the first major axis (412j) substantially parallel to a machine direction of the film and coinciding with the second minor axis (414n), the second major axis (414j) substantially parallel to a transverse direction orthogonal to the machine direction and offset from the first minor axis (412n), and
the second side (520) comprising a plurality of apertured protuberances (522) extending at an acute angle (β) from a second land area (524) opposite the first land area (514) and aligned with the plurality of two-dimensional apertures (512).

## Patentansprüche

1. Ausbildende Struktur (400), die eine Außenoberfläche (402), die konfiguriert ist, um eine Polymerbahn aufzunehmen, eine Innenoberfläche (404), eine Dicke (t) zwischen der Außenoberfläche (402) und der Innenoberfläche (404), und eine Vielzahl von Öffnungen (410) aufweist, die sich durch die Dicke (t) in einem spitzen Winkel (β) relativ zu einer Ebene tangential zu der Außenoberfläche (402) erstrecken, wobei jede der Vielzahl von Öffnungen (410) eine Form an der Außenoberfläche (402) aufweist, die durch eine erste Ellipse (412), die eine erste Hauptachse (412j) und eine erste Nebenachse (412n) aufweist, und eine zweite Ellipse (414) definiert ist, die eine zweite Hauptachse (414j) und eine zweite Nebenachse (414n) aufweist, wobei die erste Hauptachse (412j) im Wesentlichen parallel zu einer Maschinenrichtung ist und mit der zweiten Nebenachse (414n) zusammenfällt, wobei die zweite Hauptsache (414j) im Wesentlichen parallel zu einer Querrichtung orthogonal zu der Maschinenrichtung ist und von der ersten Nebenachse (412n) versetzt ist.

2. Ausbildende Struktur (400) nach Anspruch 1, wobei der spitze Winkel (β) zwischen etwa 15° und etwa 45° liegt.

3. Ausbildende Struktur (400) nach Anspruch 2, wobei der spitze Winkel (β) zwischen etwa 20° und etwa 40° liegt.

4. Ausbildende Struktur (400) nach Anspruch 1, wobei Mitten angrenzender Öffnungen (410) äquidistant voneinander beabstandet sind.

5. Ausbildende Struktur (400) nach Anspruch 1, wobei jede der Vielzahl von Öffnungen (410) eine Form an der Innenoberfläche (404) aufweist, die gleich der Form an der Außenoberfläche (402) ist.

6. Ausbildende Struktur (400) nach Anspruch 1, wobei die Dicke (t) der ausbildenden Struktur eine Vielzahl von Schichten umfasst, wobei jede Schicht relativ zu einer angrenzenden Schicht gestaffelt ist, um die Vielzahl von Öffnungen durch die Dicke (t) in dem spitzen Winkel (β) zu definieren.

7. Vorrichtung (200) zum Ausbilden einer atmungsaktiven, flüssigkeitsundurchlässigen, mit Löchern versehenen ausgebildeten Folie, die Vorrichtung umfassend:
die ausbildende Struktur (400) nach einem der Ansprüche 1 bis 6; und
ein Vakuumsystem (210), das konfiguriert ist, um ein Vakuum an der Innenoberfläche (404) der ausbildenden Struktur zu erzeugen und Abschnitte der Polymerbahn in die Vielzahl von Öffnungen (410) zu ziehen.

8. Verfahren zum Ausbilden einer atmungsaktiven, flüssigkeitsundurchlässigen, mit Löchern versehenen ausgebildeten Folie (400), das Verfahren umfassend:
Inberührungbringen einer Polymerbahn (204) mit der ausbildenden Struktur (400) nach einem der Ansprüche 1 bis 6; und
Anlegen eines Vakuums an die Innenoberfläche (404) der ausbildenden Struktur, um Abschnitte der Polymerbahn (204) in die Vielzahl von Öffnungen (410) zu ziehen und eine Vielzahl von mit Löchern versehenen Erhebungen zu erzeugen.

9. Atmungsaktive, flüssigkeitsundurchlässige, mit Löchern versehene ausgebildete Folie (500), umfassend:
eine erste Seite (510) und eine zweite Seite (520) gegenüber der ersten Seite (510);
die erste Seite (510) umfassend einen ersten Stegabschnitt (514) und eine Vielzahl von zweidimensionalen Löchern (512), wobei jedes der Vielzahl von zweidimensionalen Löchern (512) eine Form aufweist, die durch eine erste Ellipse (412), die eine erste Hauptachse (412j) und eine erste Nebenachse (412n) aufweist, und eine zweite Ellipse (414) definiert ist, die eine zweite Hauptachse (414j) und eine zweite Nebenachse (414n) aufweist, wobei die erste Hauptachse (412j) im Wesentlichen parallel zu einer Maschinenrichtung der Folie ist und mit der zweiten Nebenachse (414n) zusammenfällt, wobei die zweite Hauptachse (414j) im Wesentlichen parallel zu einer Querrichtung orthogonal zu der Maschinenrichtung ist und von der ersten Nebenachse (412n) versetzt ist, und
die zweite Seite (520) umfassend eine Vielzahl von mit Löchern versehenen Erhebungen (522), die sich in einem spitzen Winkel (B) von einem zweiten Stegabschnitt (524) gegenüber dem ersten Stegabschnitt (514) erstrecken und an der Vielzahl von zweidimensionalen Löchern (512) ausgerichtet sind.

## Revendications

1. Structure de formage (400) ayant une surface externe (402) conçue pour recevoir une bande de polymère, une surface interne (404), une épaisseur (t) entre la surface externe (402) et la surface interne (404), et une pluralité d'ouvertures (410) s'étendant à travers l'épaisseur (t) à un angle aigu (β) par rapport à un plan tangentiel à la surface externe (402), chacune parmi la pluralité d'ouvertures (410) ayant une forme au niveau de la surface externe (402) définie par une première ellipse (412) ayant un premier axe principal (412j) et un premier axe mineur (412n), et une seconde ellipse (414) ayant un second axe principal (414j) et un second axe mineur (414n), le premier axe principal (412j) sensiblement parallèle à une direction de machine et coïncidant avec le second axe mineur (414n), le second axe principal (414j) sensiblement parallèle à une direction transversale orthogonale à la direction de la machine et décalé du premier axe mineur (412n).

2. Structure de formage (400) selon la revendication 1, dans laquelle l'angle aigu (β) est compris entre environ 15 ° et environ 45 °.

3. Structure de formage (400) selon la revendication 2, dans laquelle l'angle aigu (β) est compris entre environ 20 ° et environ 40 °.

4. Structure de formage (400) selon la revendication 1, dans laquelle des centres d'ouvertures adjacentes (410) sont espacés équidistants les uns des autres.

5. Structure de formage (400) selon la revendication 1, dans laquelle chacune parmi la pluralité d'ouvertures (410) a une forme au niveau de la surface interne (404) la même que la forme au niveau de la surface externe (402).

6. Structure de formage (400) selon la revendication 1, dans laquelle l'épaisseur (t) de la structure de formage comprend une pluralité de couches, chaque couche étant décalée par rapport à une couche adjacente pour définir la pluralité d'ouvertures à travers l'épaisseur (t) à l'angle aigu (β).

7. Appareil (200) de formation d'un film formé perforé, imperméable aux liquides, respirant, l'appareil comprenant :
la structure de formage (400) selon l'une quelconque des revendications 1 à 6 ; et
un système de vide (210) conçu pour créer un vide au niveau de la surface interne (404) de la structure de formage et tirer des parties de la bande de polymère dans la pluralité d'ouvertures (410).

8. Procédé permettant de former un film formé perforé, imperméable aux liquides et respirant (400), le procédé comprenant les étapes consistant à :
la mise en contact d'une bande de polymère (204) avec la structure de formage (400) selon l'une quelconque des revendications 1 à 6 ; et
l'application d'un vide à la surface intérieure (404) de la structure de formage pour tirer des parties de la bande de polymère (204) dans la pluralité d'ouvertures (410) et créer une pluralité de protubérances perforées.

9. Film formé perforé, imperméable aux liquides et respirant (500), comprenant :
un premier côté (510) et un second côté (520) opposé au premier côté (510) ;
le premier côté (510) comprenant une première zone de méplat (514) et une pluralité d'ouvertures bidimensionnelles (512), chacune parmi la pluralité d'ouvertures bidimensionnelles (512) ayant une forme définie par une première ellipse (412) ayant un premier axe principal (412j) et un premier axe mineur (412n), et une seconde ellipse (414) ayant un second axe principal (414j) et un second axe mineur (414n), le premier axe principal (412j) sensiblement parallèle à une direction de machine du film et coïncidant avec le second axe mineur (414n), le second axe principal (414j) sensiblement parallèle à une direction transversale orthogonale à la direction de la machine et décalé du premier axe mineur (412n), et
le second côté (520) comprenant une pluralité de protubérances ouvertes (522) s'étendant à un angle aigu (β) à partir d'une seconde zone de méplat (524) opposée à la première zone de méplat (514) et alignées avec la pluralité d'ouvertures bidimensionnelles (512).
